# FASCICULE DE BREVET EUROPEEN

(11) **EP 4 251 400 B1**
(45) Date de publication et mention de la délivrance du brevet: **14.08.2024**
(21) Numéro de dépôt: 21816060.4
(22) Date de dépôt: 24.11.2021
(51) Int. Cl.: B29C 49/42, B29C 49/46, B08B 3/00, B08B 9/032, B29C 49/60, A61L 2/20

(54) **MACHINE DE FORMAGE COMPORTANT UN DISPOSITIF DE STÉRILISATION D'UN RÉSEAU DE SOUFFLAGE**
UMFORMMASCHINE MIT EINER VORRICHTUNG ZUM STERILISIEREN EINES BLASNETZWERKS
FORMING MACHINE COMPRISING A DEVICE FOR STERILISING A BLOWING NETWORK

(30) Priorité: 26.11.2020 FR 2012171
(43) Date de publication de la demande: 04.10.2023
(73) Titulaire: Sidel Participations, 76930 Octeville-sur-Mer (FR)
(72) Inventeur: MARIE, Jérémy, 76930 Octeville-sur-Mer (FR); TOUTAIN, Grégory, 76930 Octeville-sur-Mer (FR); LETELLIER, Sandy, 76930 Octeville-sur-Mer (FR)
(74) Mandataire: Sidel Group
(86) Numéro de dépôt international: PCT/EP2021/082776
(87) Numéro de publication internationale: WO 2022/112301

(56) Documents cités:
- EP-A2- 2 388 126
- WO-A1-2007/142795
- DE-A1- 102013 101 642

## Description

### Domaine technique de l'invention

L'invention se rapporte à une machine de formage pour le formage de récipients à partir de préformes en matériau thermoplastique, la machine de formage comportant un réseau de soufflage qui comporte des conduites qui raccordent une source commandée de gaz de soufflage comprimé à au moins une tuyère de soufflage pour former les récipients par soufflage, la machine de formage comportant un dispositif de stérilisation du réseau de soufflage comportant :
- une source de liquide stérilisant comportant un agent oxydant ;
- un évaporateur dans lequel de la vapeur stérilisante est produite par évaporation totale du liquide stérilisant fourni par la source de liquide stérilisant ;
- un dispositif de distribution du liquide stérilisant qui est interposé entre la source de liquide stérilisant et l'évaporateur.

Des dispositifs apparentés et selon l'art antérieur sont décrits dans les documents DE102013-101642A1, EP2388126A2 et WO2007/142795A1.

### Arrière-plan technique

Les récipients en matériau thermoplastique, tels que des bouteilles, flacons ou pots, sont généralement obtenus à partir de préformes qui sont préalablement fabriquées par moulage par injection de la matière thermoplastique, telle que du PET (PolyEthylène Terephtalate), et qui sont ultérieurement chauffées pour obtenir des préformes chaudes aptes au moulage.

Pour ce faire, une installation de fabrication de récipients comporte au moins un four pour chauffer des préformes qui est associé à une machine de formage (aussi appelée « souffleuse ») pour le moulage des récipients à partir desdites préformes chaudes. L'installation comporte encore avantageusement une unité de stérilisation pour stériliser au moins l'intérieur des préformes de manière à obtenir des récipients dits « stériles ».

Pour réaliser le moulage par soufflage ou par étirage-soufflage d'une préforme chaude, en une ou plusieurs étapes, il est connu d'utiliser pour le soufflage au moins un fluide, généralement un gaz, tel que de l'air comprimé à des pressions pouvant atteindre des valeurs de 30 ou 40 bars selon les applications. Pour permettre de répartir la matière de manière plus homogène dans le récipient final, il est parfois procédé à une opération préliminaire de présoufflage qui consiste à injecter un fluide de soufflage à plus basse pression, par exemple à 13 bars, dans la préforme avant de procéder à l'opération de soufflage proprement dite au moyen d'un gaz de soufflage à haute pression comme expliqué précédemment.

L'air utilisé pour le soufflage est introduit à l'intérieur de la préforme chaude placée dans un moule de sorte que l'air de soufflage entre directement en contact avec la surface interne du récipient. A cet effet, la machine de formage comporte une source d'air de soufflage qui alimente plusieurs tuyères de soufflage par l'intermédiaire de conduites de soufflage.

Or, la surface interne est elle-même destinée à être ultérieurement en contact avec le produit qui sera conditionné dans le récipient.

La qualité de l'air de soufflage, plus particulièrement l'absence de contaminants, tels que des micro-organismes, des particules comme des poussières, etc., est donc un paramètre important à prendre en compte dans la maîtrise globale des risques de contamination des récipients fabriqués et ceci afin de pouvoir garantir, notamment dans le cas de conditionnement agro-alimentaire, une bonne conservation du produit conditionné, notamment une durée de conservation, et la sécurité du consommateur.

Or, la qualité de l'air comprimé utilisé pour le soufflage est déterminée par un ensemble de facteurs, depuis la qualité de l'air atmosphérique aspiré qui varie selon l'environnement du site industriel et sa localisation par rapport à des sources polluantes, jusqu'à l'état du réseau de distribution et/ou de l'installation.

L'air atmosphérique aspiré et comprimé par au moins un compresseur présente par exemple un degré d'hygrométrie plus ou moins important or l'humidité favorise la corrosion et le développement des micro-organismes.

Une attention particulière est également portée dans le choix des compresseurs, les compresseurs étant selon leur conception, tout particulièrement les moyens de lubrification, susceptibles d'engendrer une contamination chimique de l'air par exemple par de l'huile de lubrification ou encore des poussières de téflon.

C'est la raison pour laquelle, l'air comprimé destiné à être utilisé pour le soufflage est préalablement traité et tout particulièrement filtré par des moyens de filtration de gaz afin d'obtenir un air de soufflage qui soit « stérile », c'est-à-dire notamment exempt de micro-organismes.

L'air comprimé utilisé pour le soufflage est généralement successivement filtré par un système de filtration comportant différents moyens de filtration de gaz.

De manière non limitative, un tel système de filtration d'air destiné au soufflage comporte par exemple des moyens de filtration multiples qui, agencés en série, sont destinés à délivrer en sortie un air stérile.

L'air y est par exemple successivement filtré par des premiers moyens de filtration de type « FFP » pour obtenir notamment un déshuilage, une épuration en eau et une élimination des poussières, puis des deuxièmes moyens de filtration de type « AK » (à charbons actifs) pour ôter toutes les vapeurs d'huile et d'hydrocarbures gazeux pouvant en outre entraîner une nuisance olfactive et gustative, et enfin par des troisièmes moyens de filtration de type « SRF » pour retenir les micro-organismes.

En effet, l'air de soufflage est susceptible d'être un vecteur de contamination de l'intérieur de la préforme, et donc du récipient, en y introduisant des contaminants et tout particulièrement des micro-organismes (virus, germes, spores, etc.).

La maîtrise de la qualité de l'air comprimé utilisé pour le soufflage est encore plus importante lorsque, dans le procédé de fabrication des récipients, le remplissage des récipients est réalisé dans un environnement aseptique directement après le moulage du récipient obtenu par soufflage ou par étirage-soufflage d'une préforme chaude.

En effet, dans un tel procédé de fabrication, la stérilisation est généralement effectuée en amont sur les préformes, avant leur transformation en récipients, de sorte qu'il est ensuite essentiel de prévenir les risques de contamination des préformes stérilisées, comme celle des récipients fabriqués à partir de ces préformes.

L'invention vise à stériliser les conduites de soufflage qui sont agencées en aval des moyens de filtrations et qui conduisent l'air de soufflage stérile jusqu'aux tuyères de soufflage. Ceci permet notamment de garantir que l'air demeure stérile jusqu'à ce qu'il atteigne les préformes.

La stérilisation des conduites de soufflage doit permettre de détruire les micro-organismes présents afin d'en prévenir le développement dans les conduites de soufflage et ainsi éliminer les risques de contamination des préformes.

Dans le cas d'une application industrielle comme celle de la fabrication de récipients destinés au conditionnement de produits agro-alimentaire, il est important de pouvoir garantir une qualité et tout particulièrement la stérilité de l'air de soufflage.

Pour stériliser les conduites de soufflage, il est connu de faire circuler un mélange gazeux stérilisant comportant un agent oxydant en phase gazeuse dans les conduites de soufflage. L'agent oxydant en phase vapeur est généralement formée par évaporation de l'agent oxydant en phase liquide, par exemple du peroxyde d'hydrogène (H2O2).

Pour permettre de doser précisément la quantité de liquide stérilisant présente dans le mélange gazeux, il est connu d'utiliser une pompe péristaltique. Une pompe péristaltique présente un tuyau souple qui est agencé en "U" autour d'une roue munie de patins qui écrasent localement le tuyau. Lorsque la roue tourne, les patins glissent contre le tuyau, déplaçant la zone écrasée et poussant le liquide situé en aval de cette zone vers l'évaporateur.

Une telle pompe présente en outre l'avantage d'éviter que des pièces sujettes à la corrosion ne soient exposées au liquide stérilisant.

Cependant, une telle pompe ne permet pas de distribuer le liquide stérilisant de manière stable. En effet, par nature, une pompe péristaltique distribue le liquide stérilisant avec un débit pulsé du fait de la présence des zones d'écrasement du tuyau par les patins. Ainsi, lorsqu'une consigne de débit de liquide stérilisant est communiquée à la pompe, cette dernière permet de délivrer '"en moyenne" un débit égal à la consigne, cependant, dans la réalité, le débit oscillera en permanence entre un débit supérieur à la consigne de débit et un débit inférieur à la consigne de débit. Ceci signifie que le mélange gazeux stérilisant qui circule dans les conduites de soufflage ne présente pas une concentration homogène en agent oxydant. Cela augmente le risque de condensation de l'agent oxydant sur les conduites de soufflage dans les zones présentant une plus grande concentration d'agent oxydant.

Pour résoudre ce problème l'invention propose un dispositif de distribution du liquide stérilisant qui permette un dosage précis, qui ne soit pas sujet à la corrosion et qui produise un débit stable de liquide stérilisant. Le terme "stable" est utilisé par opposition au terme pulsé. Ainsi, une pompe péristaltique n'est pas considérée comme un dispositif de distribution de liquide stérilisant qui produit un débit stable au sens de l'invention.

### Résumé de l'invention

L'invention propose une machine de formage pour le formage de récipients à partir de préformes en matériau thermoplastique, la machine de formage comportant un réseau de soufflage qui comporte des conduites qui raccordent une source commandée de gaz de soufflage comprimé à au moins une tuyère de soufflage pour former les récipients par soufflage, la machine de formage comportant un dispositif de stérilisation du réseau de soufflage comportant :
- une source de liquide stérilisant comportant un agent oxydant ;
- un évaporateur dans lequel de la vapeur stérilisante est produite par évaporation totale du liquide stérilisant fourni par la source de liquide stérilisant ;
- un dispositif de distribution du liquide stérilisant qui est interposé entre la source de liquide stérilisant et l'évaporateur ;
   caractérisé en ce que le dispositif de distribution est conçu pour délivrer un débit continu et stable de liquide stérilisant.

Selon une autre caractéristique de l'invention, le dispositif de distribution comporte :
- une première chambre à volume variable communiquant avec la source de liquide stérilisant et avec l'évaporateur, le volume de la première chambre à volume variable étant commandé par le déplacement d'un premier piston mobile alternativement dans un sens d'aspiration ou dans un sens opposé de refoulement ;
- une deuxième chambre à volume variable communiquant avec la source de liquide stérilisant et avec l'évaporateur, le volume de la deuxième chambre volume variable étant commandé par le déplacement d'un deuxième piston mobile alternativement dans un sens d'aspiration ou dans un sens opposé de refoulement, ;
- un dispositif de commande du déplacement du premier piston et du deuxième piston de manière qu'une des deux chambres à volume variable aspire le liquide stérilisant pendant que l'autre des deux chambres à volume variable refoule le liquide stérile.

Selon une autre caractéristique de l'invention, le dispositif de distribution comporte uniquement deux chambres à volume variable.

Selon une autre caractéristique de l'invention, le dispositif de commande est conçu pour déplacer simultané les deux pistons en sens opposées.

Selon une autre caractéristique de l'invention, le rapport de la variation de volume d'une des chambres à volume variable sur la distance de déplacement du piston associé est identique pour la première chambre à volume variable et pour la deuxième chambre à volume variable.

Selon une autre caractéristique de l'invention, le dispositif de commande déplace le premier piston et le deuxième piston simultanément à la même vitesse dans deux sens opposés.

Selon une autre caractéristique de l'invention, les deux pistons sont liés mécaniquement en déplacement l'un à l'autre, le dispositif de commande comportant un unique actionneur commun aux deux pistons.

Selon une autre caractéristique de l'invention, les deux pistons sont fixés ensemble.

Selon une autre caractéristique de l'invention, les deux pistons sont commandés indépendamment par deux actionneurs individuels.

Selon une autre caractéristique de l'invention, les deux actionneurs individuels sont asservis l'un à l'autre.

Selon une autre caractéristique de l'invention, les chambres à volume variable sont raccordées en parallèle à la source de liquide stérilisant, chacune par une vanne commandée associée.

Selon une autre caractéristique de l'invention, les chambres à volume variable sont raccordées en parallèle à la source de liquide stérilisant par un clapet anti-retour qui autorise la circulation du liquide stérilisant uniquement depuis la source de liquide stérilisant vers la chambre à volume variable associée.

Selon une autre caractéristique de l'invention, les chambres à volume variable sont raccordées en parallèle à un système d'évacuation.

Selon une autre caractéristique de l'invention, les chambres à volume variable sont raccordées en parallèle à l'évaporateur, chacune par l'intermédiaire d'une vanne 90A, 90B) commandée associée.

Selon une autre caractéristique de l'invention, chaque chambre à volume variable est raccordée au système d'évacuation par l'intermédiaire d'une vanne commandée associée.

### Brève description des figures

D'autres caractéristiques et avantages de l'invention apparaitront au cours de la lecture de la description détaillée qui va suivre pour la compréhension de laquelle on se reportera aux dessins annexés dans lesquels :
[Fig.1] La [Fig.1] est un schéma pneumatique qui représente une machine de formage pour la mise en oeuvre du procédé réalisé selon les enseignements de l'invention.
[Fig.2] La [Fig.2] est un schéma pneumatique qui représente plus en détails une des stations de moulage qui équipe la machine de formage de la [Fig.1].
[Fig.3] La [Fig.3] est un schéma pneumatique qui représente plus en détails un dispositif de stérilisation qui équipe la machine de formage de la [Fig.1] ;
[Fig.4] La [Fig.4] est un schéma pneumatique qui représente en détails un dispositif de distribution de liquide stérilisant qui est réalisé selon un premier mode de réalisation de l'invention et qui équipe le dispositif de stérilisation de la [Fig.3].
[Fig.5] La [Fig.5] est un schéma pneumatique qui représente en détails un dispositif de distribution de liquide stérilisant qui est réalisé selon un deuxième mode de réalisation de l'invention et qui équipe le dispositif de stérilisation de la [Fig.3].

### Description détaillée de l'invention

Dans la suite de la description, des éléments présentant une structure identique ou des fonctions analogues seront désignés par une même référence.

Dans la suite de la description, les termes "amont" et "aval" sont utilisés pour décrire le sens d'écoulement d'un gaz dans des conduites.

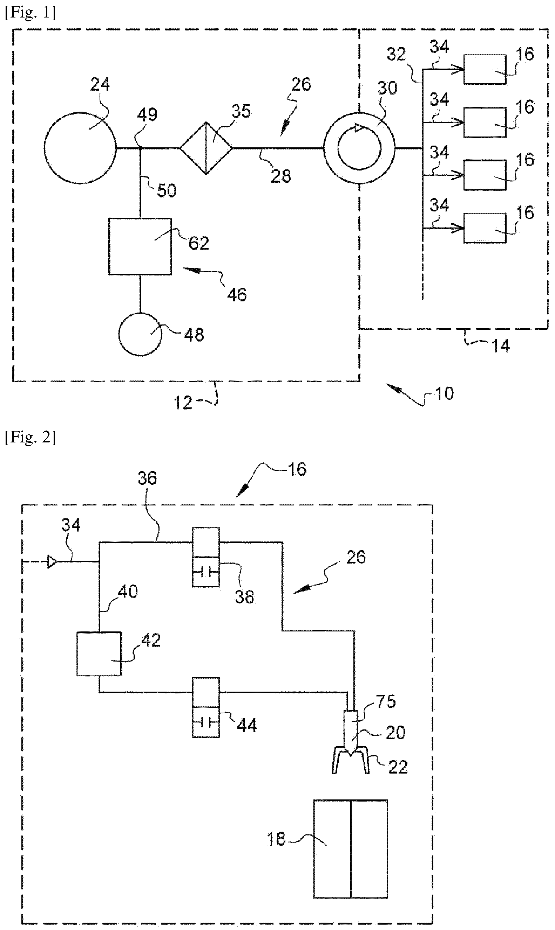

On a représenté à la [Fig.1] un schéma pneumatique illustrant un exemple de réalisation d'une machine 10 de formage de récipients par soufflage ou étirage-soufflage de préforme en matériau thermoplastique.

La machine 10 de formage présente une partie 12 fixe par rapport au sol et une partie 14 tournante qui est montée sur un carrousel tournant (non représenté).

La partie 14 tournante comporte plusieurs stations 16 de moulage qui sont embarquées sur le carrousel. Cette disposition permet de produire des récipients en grandes séries. Comme représenté à la [Fig.2], chaque station 16 de moulage comporte un moule 18 qui est destiné à recevoir une préforme et qui présente une empreinte du récipient à produire. Chaque station 16 de moulage comporte aussi une tuyère 20 de soufflage qui est conformée de manière à insuffler un gaz de formage sous pression dans la préforme reçue dans le moule 18. Il s'agit par exemple d'une tuyère 20 qui comporte un embout 22 en forme de cloche qui est destiné à venir coiffer un col de la préforme reçue dans le moule 18.

La partie 12 fixe comporte une source 24 commandée de gaz de soufflage comprimé. Le gaz de soufflage est par exemple comprimé à environ 40 bars. Le gaz de soufflage est par exemple de l'air.

La source 24 commandée de gaz de soufflage est raccordée aux tuyères 20 de chaque stations 16 de moulage par un réseau de conduites soufflage, dit réseau 26 de soufflage dans la suite de la description et dans les revendications. Les conduites du réseau 26 de soufflage sont ici réalisées dans un matériau qui est susceptible d'être oxydé rapidement au contact d'un agent oxydant sous forme liquide, tel que du peroxyde d'hydrogène ou de l'acide acétique, contrairement à des conduites réalisées en matériau inoxydable, tel que de l'acier inoxydable.

Plus particulièrement le réseau 26 de soufflage comporte une conduite 28 principale d'alimentation qui raccorde la source 24 commandée de gaz de soufflage à un joint 30 tournant qui permet de faire l'interface entre la partie 12 fixe et la partie 14 tournante. Le réseau 26 de soufflage comporte aussi au moins une rampe 32 de distribution qui appartient à la partie 14 tournante et qui est raccordée à la conduite 28 principale d'alimentation par l'intermédiaire du joint 30 tournant. La rampe 32 de distribution est raccordée à chacune des stations 16 de moulage au moyens de conduites 34 de distribution associées.

Pour garantir la qualité de l'air de soufflage, un organe 35 de filtration, par exemple un filtre de type "SRF", est interposé dans la conduite 28 principale d'alimentation.

Comme représenté à la [Fig.2], dans chacune des stations 16 de moulage, le réseau 26 de soufflage comporte aussi une conduite 36 de soufflage haute pression qui raccorde la conduite 34 de distribution associée à la tuyère 20. Une vanne 38 de soufflage à deux voies est interposée dans la conduite 36 de soufflage haute pression. La vanne 38 de soufflage est commandée alternativement entre une position ouverte et une position fermée.

Dans chacune des stations 16 de moulage, le réseau 26 de soufflage comporte aussi une conduite 40 de présoufflage basse pression qui raccorde la conduite 34 de distribution associée à la tuyère 20 en parallèle avec la conduite 36 de soufflage haute pression. La conduite 40 de soufflage basse pression comporte un organe 42 de régulation de la pression du gaz de soufflage afin d'en faire baisser la pression, par exemple jusqu'à environ 13 bars. Une vanne 44 de présoufflage à deux voies est interposée dans la conduite 40 de soufflage basse pression. La vanne 44 de présoufflage est commandée alternativement entre une position ouverte et une position fermée.

La machine 10 de formage comporte aussi un dispositif 46 de stérilisation du réseau 26 de soufflage. Le dispositif 46 de stérilisation comporte une source 48 commandée d'un gaz de séchage chaud sous pression, par exemple de l'air. La source 48 commandée de gaz de séchage est distincte de la source 24 commandée de gaz de soufflage. La source 48 commandée de gaz de séchage est raccordée au réseau 26 de soufflage en un embranchement 49 d'alimentation par l'intermédiaire d'une conduite 50 de stérilisation.

La source 48 commandée de gaz de séchage est ici agencée sur la partie 12 fixe de la machine 10 de formage. L'embranchement 49 d'alimentation est de préférence aussi agencé sur la partie 12 fixe de la machine 10 de formage, en amont du joint 30 tournant. L'embranchement 49 d'alimentation est ici agencé dans la conduite 28 principale de soufflage. L'embranchement 49 d'alimentation est plus particulièrement agencé en amont de l'organe 35 de filtration.

En variante non représentée de l'invention, l'embranchement d'alimentation est agencé en aval mais à proximité de l'organe de filtration. Dans ce cas, il est préférable de prévoir des moyens de stérilisation supplémentaires pour l'organe de stérilisation.

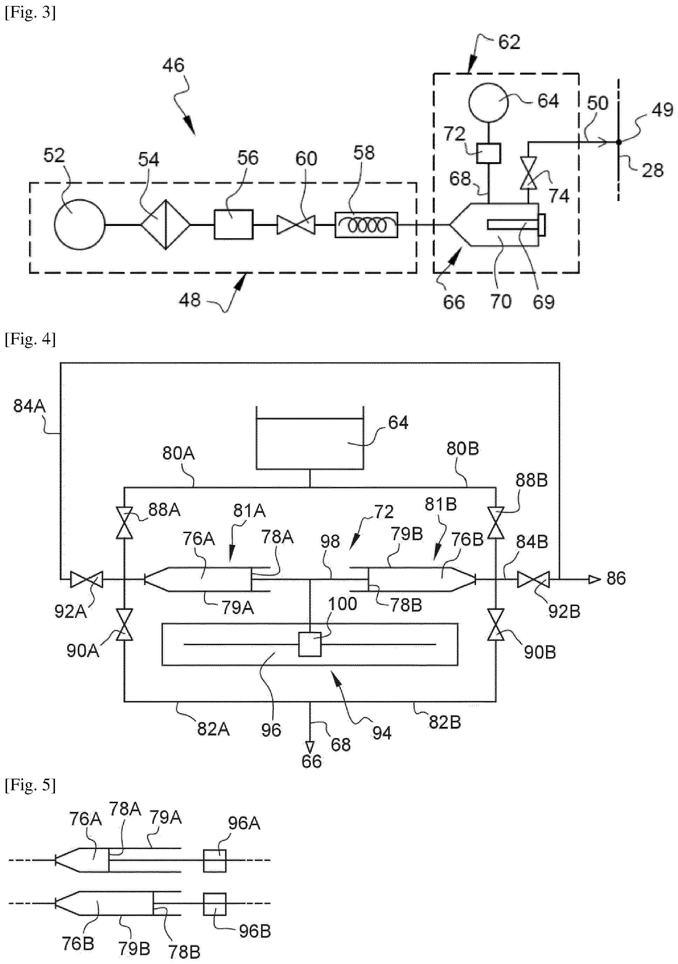

On a représenté plus en détails à la [Fig.3] le dispositif 46 de stérilisation. La source 48 commandée de gaz de séchage comporte une source 52 de gaz sous pression, par exemple de l'air, qui alimente la conduite 50 de stérilisation. La source 48 commandée de gaz de séchage comporte en outre un filtre 54 pour garantir que le gaz de séchage ne comporte aucune impureté, un organe 56 de régulation de la pression, et un organe 58 de chauffage du gaz de séchage à une température déterminée. La température "Tg" du gaz de séchage chaud est par exemple d'environ 180°C. La source 48 commandée de gaz de séchage comporte en outre une vanne 60 de régulation du débit de gaz de séchage. La vanne 60 de régulation est ici commandée dans une position fermée dans laquelle le débit de gaz de séchage est nul ou dans une position ouverte dans laquelle le gaz de séchage est délivré avec un débit constant.

Le dispositif 46 de stérilisation comporte aussi une source 62 commandée de vapeur stérilisante. Comme représenté à la [Fig.3], elle comporte un réservoir 64 de liquide stérilisant comportant un agent oxydant. L'agent oxydant est par exemple du peroxyde d'hydrogène (H2O2) ou de l'acide peracétique. Le liquide stérilisant est par exemple formé d'une solution aqueuse comportant une concentration déterminée d'agent oxydant, par exemple une solution de peroxyde d'hydrogène dilué à 25% dans de l'eau.

La source 62 commandée de vapeur stérilisante comporte aussi un évaporateur 66 dans lequel de la vapeur stérilisante est produite par évaporation totale du liquide stérilisant fourni par le réservoir 64 de liquide stérilisant par l'intermédiaire d'une conduite 68 d'injection. L'évaporateur 66 comporte un organe 69 chauffant à une température suffisante pour permettre l'évaporation instantanée du liquide stérilisant. L'organe 69 chauffant est agencé dans une chambre de l'évaporateur 66. La vapeur stérilisante comporte ainsi de l'agent oxydant vaporisé et de la vapeur d'eau dans des proportions bien définies.

La source 62 commandée de vapeur stérilisante comporte en outre un dispositif 72 de distribution du liquide stérilisant avec un débit réglable qui est interposé entre le réservoir 64 de liquide stérilisant et l'évaporateur 66.

La chambre de l'évaporateur 66 forme ici une chambre 70 de mélange de la vapeur stérilisante avec le gaz de séchage pour former un mélange gazeux stérilisant présentant une pression partielle de vapeur stérilisante déterminée en fonction du débit massique de gaz de séchage et du débit massique de vapeur stérilisante. A cet effet, la chambre 70 de mélange est interposée dans la conduite 50 de stérilisation.

La chambre 70 de mélange étant ici formée directement par la chambre de l'évaporateur 66, le débit massique de vapeur stérilisante est compris comme étant la masse de vapeur stérilisante produite par évaporation dans l'évaporateur 66 par unité de temps.

En variante non représentée, la chambre de mélange est distincte de la chambre de l'évaporateur. La chambre de mélange est alors agencée en aval de l'évaporateur selon le sens d'écoulement de la vapeur stérilisante.

Le dispositif 46 de stérilisation comporte aussi une vanne 74 de régulation du débit qui est interposée dans la conduite 50 de stérilisation en aval de la chambre 70 de mélange. La vanne 74 de régulation est ici commandée soit dans une position fermée dans laquelle elle interdit le passage de tout gaz vers le réseau 26 de soufflage, soit dans une position ouverte.

La machine 10 de formage est susceptible de fonctionner selon un mode de production dans lequel le dispositif 46 de stérilisation est commandée de manière à n'émettre ni vapeur stérilisante, ni gaz de séchage dans le réseau 26 de soufflage. En mode de production; la source 24 commandée de gaz de soufflage est commandée de manière à injecter du gaz de soufflage dans le réseau 26 de soufflage. La vanne 38 de soufflage et la vanne 44 de présoufflage de chaque station 16 de moulage sont commandées en fonction de la position de ladite station 16 de moulage par rapport à la partie 12 fixe pour insuffler tour à tour du gaz de soufflage à basse pression puis du gaz de soufflage à haute pression pour permettre le formage du récipient à partir d'une préforme.

La machine 10 de formage est aussi susceptible de fonctionner selon un mode de stérilisation qui ne peut être mis en oeuvre que lorsque le mode de production est désactivé. Le mode de stérilisation est par exemple activé lors d'un changement de format de récipient et/ou encore à intervalle de temps régulier pour éviter le développement de germes dans le réseau 26 de soufflage. Lors de la mise en oeuvre du mode de stérilisation, le moule est vide, c'est-à-dire qu'il ne comporte aucune préforme.

Préalablement au commencement du procédé de stérilisation, une consigne de débit de vapeur stérilisante est déterminée de manière à garantir une stérilisation satisfaisante du réseau 26 de soufflage. Cette consigne est par exemple fonction de la longueur du réseau 26 de soufflage, et notamment du nombre de stations 16 de moulage. On détermine aussi une durée "D" d'exposition du réseau 26 de soufflage au mélange gazeux stérilisant.

Pour permettre la stérilisation de la totalité des conduites du réseau 26 de soufflage, il est prévu d'ouvrir les vannes 38 de soufflage et les vannes 44 de présoufflage pour toute la durée du procédé de stérilisation comme cela est représenté à la [Fig.2]. La source 24 commandée de gaz de soufflage est commandée en position fermée pour toute la durée du procédé de stérilisation.

Le procédé comporte une première étape "E1" de chauffage des conduites du réseau 26 de soufflage. Lors de cette première étape "E1", seul le gaz de séchage chaud sous pression est injecté dans le réseau 26 de soufflage avec le débit déterminé. Le gaz de séchage chauffe ainsi chaque conduite du réseau 26 de soufflage au moins jusqu'à une température "Ti" de traitement par écoulement dans un sens unique depuis l'embranchement 49 d'alimentation jusqu'à la tuyère 20.

Le procédé comporte aussi une deuxième étape "E2" d'injection du mélange gazeux stérilisant dans les conduites de soufflage, qui est déclenchée à l'issue de la première étape "E1" de chauffage. Le dispositif 72 de distribution du liquide stérilisant est commandé pour injecter le liquide stérilisant avec un débit correspondant à la consigne de débit de vapeur stérilisante. Au cours de cette étape, l'agent oxydant vaporisé présent dans le mélange gazeux stérilisant stérilise les conduites du réseau 26 de soufflage par contact direct. Le mélange gazeux stérilisant s'écoule dans un sens unique depuis l'embranchement 49 d'alimentation jusqu'à la tuyère 20. Il est prévu une conduite (non représentés) d'aspiration du mélange gazeux stérilisant qui est agencé en val de la tuyère 20 ou qui est raccordé à la tuyère à proximité de l'embout 22 pour permettre d'évacuer le mélange gazeux stérilisant comportant l'agent stérilisant après son passage par le réseau 26 de soufflage.

Enfin, une troisième et dernière étape "E3" d'aération est déclenchée lorsque la durée "D" d'exposition est atteinte, comme indiqué par la condition "C3". Le dispositif 72 de distribution du liquide stérilisant est alors fermé pour interrompre la production de vapeur stérilisante. Le débit de gaz de séchage est en revanche maintenu. Le gaz de séchage continu donc à circuler, seul, dans les conduites du réseau 26 de soufflage le temps nécessaire pour évacuer les résidus de vapeur stérilisante. Le procédé est alors terminé, comme indiqué par la référence "S2".

Il est préférable que le dispositif 72 de distribution du liquide stérilisant présente un débit variable pour plusieurs raisons qui sont expliquées par la suite.

Lorsque la consigne de débit de vapeur stérilisante et la durée "D" d'exposition sont fixées préalablement. Il s'agit par exemple de valeurs constantes qui sont déterminées expérimentalement ou par calcul en fonction de caractéristiques de la machine 10 de formage, tel que le nombre de stations 16 de moulage. Même si la consigne de débit de vapeur stérilisante demeure la même pour toute la durée de la deuxième étape "E2" d'injection, il est avantageux de pouvoir installer le même dispositif 72 de distribution sur tous les modèles de machines 10 de formage pour lesquels la longueur du réseau 26 de soufflage est susceptible de varier, notamment en fonction du nombre de stations 16 de moulage. Il est donc avantageux de pouvoir faire varier le débit du dispositif 72 de distribution pour l'adapter à chacun de ces modèles. Ainsi, un premier modèle de machine 10 de formage peut comporter seulement six stations 16 de moulage, tandis qu'un deuxième modèle de machine 10 de formage peut comporter jusqu'à vingt-six stations 16 de moulage. En bénéficiant de la possibilité d'installer le même dispositif 72 de distribution sur chacun de ces modèles, cela réduit les coûts de fabrication et de stockage des dispositifs 72 de distribution.

En outre, lorsque la consigne de débit de vapeur stérilisante est susceptible de varier au cours de la deuxième étape "E2" du procédé de stérilisation sur une machine 10 de soufflage donnée, il est indispensable d'agencer un dispositif 72 de distribution à débit variable.

Par ailleurs, lors de la première étape, le réseau 26 de soufflage est chauffé au moins jusqu'à une température "Ti" de traitement déterminée. A cette température "Ti" de traitement correspond une pression de vapeur saturante pour le mélange de vapeur d'eau et d'agent oxydant vaporisé contenu dans le mélange gazeux stérilisant. La consigne de débit de vapeur stérilisante est calculée de manière que le rapport entre la pression partielle de vapeur d'eau et d'agent oxydant divisé par la pression de vapeur saturante soit proche de 90%.

Si le débit de vapeur stérilisante dépasse la valeur de consigne, il existe un risque d'atteindre un rapport de 100% ce qui se traduirait par un début de condensation du mélange de vapeur d'eau et d'agent oxydant vaporisé. Or, une telle condensation provoquerait une corrosion rapide des conduites du réseau 26 de soufflage lorsqu'elles ne sont pas réalisées dans un matériau inoxydable. Un tel matériau inoxydable étant très onéreux, l'invention propose un dispositif 72 de distribution qui garantit que la consigne de débit de vapeur stérilisante ne sera pas dépassée tout en permettant une stérilisation efficace. Ceci permet notamment de réduire très sensiblement le coût de fabrication du réseau 26 de soufflage en ne recourant pas à l'utilisation de matériau inoxydable très onéreux.

En outre, on a constaté que l'évacuation des condensats notamment sous forme liquide dans le réseau de soufflage était complexe. Les condensats ont notamment tendance à s'accumuler dans des coudes formés dans des conduites du réseau de soufflage. De ce fait, l'accumulation de condensat risque de provoquer une injection d'agent oxydant dans les premiers récipients formés par la machine de formage lors du début de production faisant suite à sa stérilisation. Ces premiers récipients sont donc généralement jetés. Le fait d'éviter la condensation pendant le procédé de stérilisation permet avantageusement d'éviter d'injecter de l'agent stérilisant dès le premier récipient produit après la stérilisation de la machine de formage.

Pour que la source 62 commandée de vapeur stérilisante soit commandée dans un débit constamment égal à la consigne lors de la deuxième étape "E2" du procédé de stérilisation, le dispositif 72 de distribution est conçu pour délivrer un débit continu et stable de liquide stérilisant pour une consigne donnée.

Un tel dispositif 72 de distribution est par exemple formé par une vanne à débit réglable. Une telle vanne serait satisfaisante pour une machine 10 de formage d'un modèle déterminé.

Cependant, une telle vanne ne permet pas toujours d'obtenir à la fois un débit précis et une amplitude de débits qui permettrait d'équiper tous les modèles de machine 10 de formage.

L'invention propose un dispositif 72 de distribution permettant d'obtenir un débit précis, stable et réglable sur une grande amplitude.

Les différents composants du dispositif 72 de distribution qui sont destinés à être en contact avec le liquide stérilisant sont de préférence réalisés en un matériau qui ne se corrode pas ou peu au contact de l'agent oxydant, par exemple en métal inoxydable et/ ou en plastique et/ou en verre.

Le dispositif 72 de distribution comporte deux chambres 76A, 76B à volume variable, et uniquement deux chambres 76A, 76B à volume variable impliquées dans le dosage et l'injection du liquide stérilisant.

Comme cela est représenté à la [Fig.4], le dispositif 72 de distribution comporte une première chambre 76A à volume variable. Le volume de la première chambre 76A à volume variable est commandé par le coulissement d'un premier piston 78A mobile dans un premier cylindre 79A. Le premier cylindre 79A et le premier piston 78A forment ainsi un premier vérin 81A.

Le premier piston 78A coulisse alternativement dans un sens d'aspiration dans lequel le volume de la première chambre 76A à volume variable augmente ou dans un sens opposé de refoulement dans lequel le volume de la première chambre 76A à volume variable diminue.

Le volume de la première chambre 76A à volume variable est ainsi susceptible de varier entre un volume minimal, correspondant à une position extrême de refoulement du piston 78A, et un volume maximal, correspondant à une position extrême d'aspiration du piston 78A.

La première chambre 76A à volume variable communique avec le réservoir 64 de liquide stérilisant par l'intermédiaire d'une première conduite 80A d'aspiration. Elle communique aussi avec l'évaporateur 66 par l'intermédiaire d'une première conduite 82A de distribution. La première chambre 76A à volume variable est aussi raccordée à une première conduite 84A de vidange qui évacue le liquide stérilisant sans passer par l'évaporateur directement vers un système 86 d'évacuation.

La première chambre 76A à volume variable est raccordée au réservoir 64 de liquide stérilisant par l'intermédiaire d'une première vanne 88A d'aspiration qui est ici interposée dans la première conduite 80A d'aspiration. La première vanne 88A d'aspiration est commandée alternativement entre une position ouverte et une position fermée. Il s'agit par exemple d'une vanne à tiroir, d'une vanne à boisseau ou encore d'une soupape.

En variante non représentée de l'invention, la vanne 88A d'aspiration est remplacée par un clapet anti-retour qui autorise la circulation du liquide stérilisant uniquement depuis le réservoir 64 de liquide stérilisant vers la première chambre 76A à volume variable lorsque le premier piston 78A est déplacé dans le sens d'aspiration.

La première chambre 76A à volume variable est raccordée à l'évaporateur 66 par l'intermédiaire d'une première vanne 90A de distribution qui est ici interposée dans la première conduite 82A de distribution. La première vanne 90A de distribution est commandée alternativement entre une position ouverte et une position fermée. Il s'agit par exemple d'une vanne à tiroir, d'une vanne à boisseau ou encore d'une soupape.

La première chambre 76A à volume variable est aussi raccordée au système 86 d'évacuation par l'intermédiaire d'une première vanne 92A d'évacuation qui est ici interposée dans la première conduite 84A de vidange. La première vanne 92A d'évacuation est commandée alternativement entre une position ouverte et une position fermée. Il s'agit par exemple d'une vanne à tiroir, d'une vanne à boisseau ou encore d'une soupape.

Le dispositif 72 de distribution comporte aussi une deuxième chambre 76B à volume variable. Le volume de la deuxième chambre 76B à volume variable est commandé par le coulissement d'un deuxième piston 78B mobile dans un deuxième cylindre 79B. Le deuxième cylindre 79B et le deuxième piston 78B forment ainsi un deuxième vérin 81B.

Le deuxième piston 78B coulisse alternativement dans un sens d'aspiration dans lequel le volume de la deuxième chambre 76B à volume variable augmente ou dans un sens opposé de refoulement dans lequel le volume de la deuxième chambre 76B à volume variable diminue.

Le volume de la deuxième chambre 76B à volume variable est ainsi susceptible de varier entre un volume minimal, correspondant à une position extrême de refoulement du piston 78B, et un volume maximal, correspondant à une position extrême d'aspiration du piston 78B.

La deuxième chambre 76B à volume variable communique avec le réservoir 64 de liquide stérilisant par l'intermédiaire d'une deuxième conduite 80B d'aspiration qui est agencée en parallèle par rapport à la première conduite 80A d'aspiration. Elle communique aussi avec l'évaporateur 66 par l'intermédiaire d'une deuxième conduite 82B de distribution qui s'étend en parallèle par rapport à la première conduite 82A de distribution. La première chambre 76A à volume variable et la deuxième chambre 76B à volume variable sont ainsi raccordées en parallèle entre le réservoir 64 de liquide stérilisant et l'évaporateur 66.

La deuxième chambre 76B à volume variable est aussi raccordée à une deuxième conduite 84B de vidange qui évacue le liquide stérilisant sans passer par l'évaporateur directement vers le système 86 d'évacuation.

La deuxième chambre 76B à volume variable est raccordée au réservoir 64 de liquide stérilisant par l'intermédiaire d'une deuxième vanne 88B d'aspiration qui est ici interposée dans la deuxième conduite 80B d'aspiration. La deuxième vanne 88B d'aspiration est commandée alternativement entre une position ouverte et une position fermée. Il s'agit par exemple d'une vanne à tiroir, d'une vanne à boisseau ou encore d'une soupape.

En variante non représentée de l'invention, la deuxième vanne 88B d'aspiration est remplacée par un clapet anti-retour qui autorise la circulation du liquide stérilisant uniquement depuis le réservoir 64 de liquide stérilisant vers la deuxième chambre 76B à volume variable lorsque le deuxième piston 78B est déplacé dans le sens d'aspiration.

La deuxième chambre 76B à volume variable est raccordée à l'évaporateur 66 par l'intermédiaire d'une deuxième vanne 90B de distribution qui est ici interposée dans la deuxième conduite 82B de distribution. La deuxième vanne 90B de distribution est commandée alternativement entre une position ouverte et une position fermée. Il s'agit par exemple d'une vanne à tiroir, d'une vanne à boisseau ou encore d'une soupape.

La deuxième chambre 76B à volume variable est aussi raccordée au système 86 d'évacuation par l'intermédiaire d'une deuxième vanne 92B d'évacuation qui est ici interposée dans la deuxième conduite 84B de vidange. La deuxième vanne 92B d'évacuation est commandée alternativement entre une position ouverte et une position fermée. Il s'agit par exemple d'une vanne à tiroir, d'une vanne à boisseau ou encore d'une soupape.

Le volume de la première chambre 76A à volume variable comprend le volume du premier cylindre 79A délimité par le premier piston 78A auquel s'ajoute le volume, dit volume mort, des tronçons de la conduite 80A d'aspiration, de la conduite 82A de distribution et de la conduite 84A de vidange qui s'étendent entre le premier cylindre 79A et les vannes 88A, 90A, 92A associées. Le volume mort est incompressible car le piston 78A ne peut pas contribuer à l'augmenter ni à le diminuer. On définit un volume mort de la même manière pour la deuxième chambre 76B à volume variable. De manière générale, les vannes 88A, 88B d'aspiration, les vannes 90A, 90B de distribution et les vannes 92A, 92B d'évacuation sont agencées au plus proche du cylindre 79A, 79B associé de manière à réduire le volume mort autant que possible.

Le dispositif 72 de distribution comporte enfin un dispositif 94 de commande du coulissement du premier piston 78A et du deuxième piston 78B de manière qu'une des deux chambres 76A, 76B à volume variable aspire le liquide stérilisant depuis le réservoir 64 de liquide stérilisant pendant que l'autre des deux chambres 76A, 76B à volume variable refoule le liquide stérilisant vers l'évaporateur 66 ou vers le système 86 d'évacuation.

Le dispositif 72 de distribution est ici conçu pour que les deux pistons 78A, 78B se déplacent simultanément dans des sens opposés, c'est-à-dire que l'un des deux pistons 78A, 78B se déplace dans un sens d'aspiration pendant que l'autre des deux pistons 78A, 78B se déplace dans un sens de refoulement.

En outre, le rapport de la variation de volume de la chambre 76A, 76B à volume variable sur la distance de déplacement du piston 78A, 78B associé est identique pour les deux chambres 76A, 76B à volume variable. Le dispositif 94 de commande est conçu pour déplacer les deux pistons 78A, 78B à la même vitesse en sens opposés. En d'autres termes, le dispositif 94 de commande permet de commander les deux pistons 78A, 78B simultanément de manière que le débit de liquide stérilisant aspiré par l'une des deux chambres 76A, 76B à volume variable soit égal au débit de liquide stérilisant refoulé par l'autre des deux chambres 76A, 76B à volume variable. Dans l'exemple représenté à la [Fig.4], les deux vérins 81A, 81B sont identiques. Ainsi, lorsque l'un des deux pistons 78A, 78B occupe sa position extrême de refoulement, l'autre des deux pistons 78A, 78B occupe sa position extrême d'aspiration, et vice versa.

Selon un premier mode de réalisation représenté à la [Fig.4], les deux pistons 78A, 78B sont liés mécaniquement en déplacement l'un à l'autre. Le dispositif 94 de commande comporte alors un unique actionneur 96 qui est commun aux deux pistons 78A, 78B.

Les deux pistons 78A, 78B sont ici fixés ensemble par l'intermédiaire d'une tige 98 commune. A cet effet, les deux vérins 81A, 81B sont agencés symétriquement l'un par rapport à l'autre. Les deux pistons 78A, 78B se déplacent ainsi selon des directions parallèle ou confondues, comme c'est ici le cas. Ainsi, lorsque les deux pistons 78A, 78B représentés à la [Fig.4] se déplacent transversalement dans la même direction, ici vers la gauche, le premier piston 78A travaille dans un sens de refoulement tandis que le deuxième piston 78B travaille dans un sens d'aspiration. L'actionneur 96 est par exemple un moteur électrique linéaire qui comporte un chariot 100 mobile qui est fixé à la tige 98.

En variante non représentée de l'invention, les deux pistons sont liés par un mécanisme comportant par exemple des tringles et/ou des engrenages.

Selon un deuxième mode de réalisation de l'invention représenté à la [Fig.5], les deux pistons 78A, 78B sont commandés indépendamment par deux actionneurs 96A, 96B individuels. Dans ce cas, les deux cylindres 79A, 79B peuvent être agencés indépendamment l'un de l'autre. Les deux actionneurs 96A, 96B individuels sont ici asservis l'un à l'autre de manière à faire bouger simultanément les deux pistons 78A, 78B dans deux sens opposés à la même vitesse.

Selon une variante de réalisation du deuxième mode de réalisation, il est possible de commander le coulissement des deux pistons de manière que l'une des chambres 76A, 76B travaillant en aspiration atteigne son volume maximal avant que l'autre des deux chambres 76A, 76B à volume variable travaillant en refoulement n'ait atteint son volume minimal.

Lors de l'utilisation du dispositif 72 de distribution, il est prévu une première phase "P1" d'amorçage qui intervient par exemple pendant la première étape "E1" de chauffage des conduites, puis une deuxième phase "P2" de distribution du liquide stérilisant à l'évaporateur 66, qui intervient pendant la deuxième étape "E2" d'injection du procédé de stérilisation, et enfin une troisième phase "P3" de vidange, qui intervient par exemple pendant l'étape "E3" d'aération du procédé de stérilisation.

Lors de la première phase "P1" d'amorçage, le réservoir 64 de liquide stérilisant est avantageusement mis sous pression afin de faciliter le remplissage des chambres 76A, 76B à volume variable et de réduire les frottements entre les pistons 78A, 78B et leur cylindre 79A, 79B associé. Le réservoir 64 de liquide stérilisant demeure sous pression au moins pendant la phase "P1" d'amorçage et pendant la phase "P2" de distribution.

Au début de cette phase "P1" d'amorçage, l'un des pistons, par exemple le premier piston 78A, occupe sa position extrême de refoulement, tandis que l'autre des pistons, ici le deuxième piston 78B, occupe sa position extrême d'aspiration. Les chambres 76A, 76B à volume variable sont de préférence remplies d'un fluide chimiquement stable, tel que de l'air.

Les pistons 78A, 78B effectuent alors un premier "aller". La première vanne 88A d'aspiration est alors commandée dans sa position ouverte, tandis que la deuxième vanne 88B d'aspiration est commandée dans sa position fermée. La première vanne 92A d'évacuation est commandée dans sa position fermée, tandis que la deuxième vanne 92B d'évacuation est commandée dans sa position ouverte. Les deux vannes 90A, 90B de distribution sont commandées en position fermée. Le dispositif 94 de commande déplace le premier piston 78A depuis sa position extrême de refoulement jusqu'à sa position extrême d'aspiration, tandis que le deuxième piston 78B est déplacé depuis sa position extrême d'aspiration jusqu'à sa position extrême de refoulement. Lors de cet "aller", la première chambre 76A à volume variable se remplit de liquide stérilisant, tandis que le fluide non corrosif contenu dans la deuxième chambre est chassé vers le système 86 d'évacuation.

Les pistons 78A, 78B effectuent ensuite un premier "retour". La deuxième vanne 88B d'aspiration est alors commandée dans sa position ouverte, tandis que la première vanne 88A d'aspiration est commandée dans sa position fermée. La deuxième vanne 92B d'évacuation est commandée dans sa position fermée, tandis que la première vanne 92A d'évacuation est commandée dans sa position ouverte. Les deux vannes 90A, 90B de distribution demeurent en position fermée. Le dispositif 94 de commande déplace le deuxième piston 78B depuis sa position extrême de refoulement jusqu'à sa position extrême d'aspiration, tandis que le premier piston 78A est déplacé depuis sa position extrême d'aspiration jusqu'à sa position extrême de refoulement. Lors de ce "retour", la deuxième chambre 76B à volume variable se remplit de liquide stérilisant, tandis que le liquide stérilisant contenu dans la deuxième chambre est chassé vers le système 86 d'évacuation.

Le déplacement des pistons 78A, 78B dans un sens puis dans l'autre sera appelé par la suite "aller-retour".

La phase "P1" d'amorçage peut comporter plusieurs "aller-retour", par exemple deux "aller-retour" afin de garantir qu'il ne demeure aucune bulle de fluide chimiquement stable, tel que de l'air, dans les chambres 76A, 76B à volume variable.

Lorsque la phase "P1" d'amorçage est terminée, la phase "P2" de distribution est enclenchée.

En début de phase "P2" de distribution, l'un des pistons, par exemple le premier piston 78A, occupe sa position extrême de refoulement, tandis que l'autre des pistons, ici le deuxième piston 78B, occupe sa position extrême d'aspiration. Les chambres 76A, 76B à volume variable sont remplies de liquide stérilisant. La première chambre 76A à volume variable présente ainsi son volume minimal tandis que la deuxième chambre 76B à volume variable présente son volume maximal. Les deux vannes 92A, 92B d'évacuation sont commandées en position fermée pour toute la durée de la phase "P2" de distribution.

Le dispositif 94 de commande est piloté par une unité électronique de commande (non représentée). Une consigne de débit de liquide stérilisant à injecter dans l'évaporateur 66 est déterminée en fonction de la consigne de débit de vapeur stérilisante par l'unité électronique de commande. La consigne de débit de liquide stérilisant est ici la même tout au long de la phase "P2" de distribution.

En variante non représentée de l'invention, la consigne de débit de liquide stérilisant peut varier au cours de la phase "P2" de distribution en fonction de plusieurs paramètres, par exemple en fonction de l'humidité relative présente à l'intérieur du réseau 26 de soufflage.

Comme cela sera expliqué par la suite, le dispositif 72 de distribution permet d'injecter le liquide stérilisant dans l'évaporateur avec un débit stable et constant qui correspond à la consigne de débit.

Les pistons 78A, 78B effectuent alors un premier "aller". La première vanne 88A d'aspiration est commandée dans sa position ouverte, tandis que la deuxième vanne 88B d'aspiration est commandée dans sa position fermée. La première vanne 90A de distribution est commandée dans sa position fermée, tandis que la deuxième vanne 90B de distribution est commandée dans sa position ouverte. Le dispositif 94 de commande déplace le premier piston 78A depuis sa position extrême de refoulement jusqu'à sa position extrême d'aspiration, tandis que le deuxième piston 78B est déplacé depuis sa position extrême d'aspiration jusqu'à sa position extrême de refoulement. La vitesse de coulissement du deuxième piston 78B est commandée de manière que le débit de liquide stérilisant injecté dans l'évaporateur 66 corresponde à la consigne de débit. Lors de cet "aller", la première chambre 76A à volume variable se remplit de liquide stérilisant, tandis que le liquide stérilisant contenu dans la deuxième chambre est injecté dans l'évaporateur 66.

Lorsque la deuxième chambre 76B à volume variable atteint son volume minimal, la première chambre 76A à volume variable occupe son volume maximal. Les pistons 78A, 78B effectuent ensuite un premier "retour". La deuxième vanne 88B d'aspiration est alors commandée dans sa position ouverte, tandis que la première vanne 88A d'aspiration est commandée dans sa position fermée. La deuxième vanne 90B de distribution est commandée dans sa position fermée, tandis que la première vanne 90A de distribution est commandée dans sa position ouverte. Le dispositif 94 de commande déplace le deuxième piston 78B depuis sa position extrême de refoulement jusqu'à sa position extrême d'aspiration, tandis que le premier piston 78A est déplacé depuis sa position extrême d'aspiration jusqu'à sa position extrême de refoulement. La vitesse de coulissement du premier piston 78A est commandée de manière que le débit de liquide stérilisant injecté dans l'évaporateur 66 corresponde à la consigne de débit. Lors de ce "retour", la deuxième chambre 76B à volume variable se remplit de liquide stérilisant, tandis que le liquide stérilisant contenu dans la deuxième chambre est injecté dans l'évaporateur 66.

Lors du changement de sens de coulissement entre un aller et un retour, ainsi qu'entre un retour et un aller, le débit de liquide stérilisant injecté dans l'évaporateur 66 demeure sensiblement stable. En effet, lorsque le changement de sens est réalisé de manière extrêmement rapide. En outre, la quantité de liquide stérilisant qui est contenu dans les conduites 82A, 82B en aval des vannes 90A, 90B de distribution est suffisante pour que le liquide stérilisant continu à s'écouler, notamment sous l'effet de la gravité, vers l'évaporateur 66 pendant le changement de sens, permettant ainsi de conserver un débit stable de liquide stérilisant. Enfin, le volume maximal des chambres 76A, 76B à volume variable est tel que très peu de changement de sens interviennent au cours de la phase "P2" de distribution au regard de la faible consigne de débit de liquide stérilisant.

A l'issue de la phase "P2" de distribution, la dernière phase "P3" de vidange est enclenchée.

En début de phase "P3" de vidange, les pistons 78A, 78B peuvent occuper une position quelconque entre leurs deux positions extrêmes. Les chambres 76A, 76B à volume variable sont remplies de liquide stérilisant. Les deux vannes 88A, 88B d'aspiration sont commandées en position fermée pour toute la durée de la phase "P2" de distribution.

Les pistons 78A, 78B effectuent alors un premier "aller". La première vanne 92A d'évacuation est commandée dans sa position fermée, tandis que la deuxième vanne 92B d'évacuation est commandée dans sa position ouverte. La première vanne 90A de distribution est commandée dans sa position ouverte, tandis que la deuxième vanne 90B de distribution est commandée dans sa position fermée. Le dispositif 94 de commande déplace le premier piston 78A depuis sa position actuelle jusqu'à sa position extrême d'aspiration, tandis que le deuxième piston 78B est déplacé depuis sa position actuelle jusqu'à sa position extrême de refoulement. Lors de cet "aller", la première chambre 76A à volume variable aspire du gaz de séchage contenu dans l'évaporateur 66, tandis que le liquide stérilisant contenu dans la deuxième chambre 76B à volume variable est vidangé vers le système 86 d'évacuation.

Lorsque la deuxième chambre 76B à volume variable atteint son volume minimal, la première chambre 76A à volume variable occupe son volume maximal. Les pistons 78A, 78B effectuent ensuite un premier "retour". La première vanne 92A d'évacuation est commandée dans sa position ouverte, tandis que la deuxième vanne 92B d'évacuation est commandée dans sa position fermée. La première vanne 90A de distribution est commandée dans sa position fermée, tandis que la deuxième vanne 90B de distribution est commandée dans sa position ouverte. Le dispositif 94 de commande déplace le premier piston 78A depuis sa position extrême d'aspiration jusqu'à sa position extrême de refoulement, tandis que le deuxième piston 78B est déplacé depuis sa position extrême de refoulement jusqu'à sa position extrême d'aspiration. Lors de ce "retour", le liquide stérilisant contenu dans la première chambre 76A à volume variable est vidangé vers le système 86 d'évacuation, tandis que la deuxième chambre 76B à volume variable aspire du gaz de séchage contenu dans l'évaporateur 66.

A la fin de cette phase "P3" de vidange, les chambres 76A, 76B à volume variable ne contiennent alors plus de liquide stérilisant et elles sont remplies de gaz de séchage. En effet, le liquide stérilisant, par exemple une solution de peroxyde d'hydrogène, est susceptible de se décomposer en eau et en oxygène s'il reste stocké trop longtemps dans le dispositif 72 de distribution, par exemple pendant que la machine 10 de formage fonctionne en mode de production.

## Revendications

1. Machine (10) de formage pour le formage de récipients à partir de préformes en matériau thermoplastique, la machine (10) de formage comportant un réseau (26) de soufflage qui comporte des conduites qui raccordent une source (24) commandée de gaz de soufflage comprimé à au moins une tuyère (20) de soufflage pour former les récipients par soufflage, la machine (10) de formage comportant un dispositif (46) de stérilisation du réseau (26) de soufflage comportant :
- une source (64) de liquide stérilisant comportant un agent oxydant; **caractérisé en ce que** la machine de formage comprenne en outre:
- un évaporateur (66) dans lequel de la vapeur stérilisante est produite par évaporation totale du liquide stérilisant fourni par la source (64) de liquide stérilisant ;
- un dispositif (72) de distribution du liquide stérilisant qui est interposé entre la source (64) de liquide stérilisant et l'évaporateur (66) ;
le dispositif (72) de distribution étant conçu pour délivrer un débit continu et stable de liquide stérilisant.

2. Machine (10) selon la revendication précédente, **caractérisé en ce que** le dispositif (72) de distribution comporte :
- une première chambre (76A) à volume variable communiquant avec la source (64) de liquide stérilisant et avec l'évaporateur (66), le volume de la première chambre (76A) à volume variable étant commandé par le déplacement d'un premier piston (78A) mobile alternativement dans un sens d'aspiration ou dans un sens opposé de refoulement, ;
- une deuxième chambre (76B) à volume variable communiquant avec la source (64) de liquide stérilisant et avec l'évaporateur (66), le volume de la deuxième chambre (76B) volume variable étant commandé par le déplacement d'un deuxième piston (78B) mobile alternativement dans un sens d'aspiration ou dans un sens opposé de refoulement, ;
- un dispositif (94) de commande du déplacement du premier piston (78A) et du deuxième piston (78B) de manière qu'une des deux chambres (76A, 76B) à volume variable aspire le liquide stérilisant pendant que l'autre des deux chambres (76B) à volume variable refoule le liquide stérile.

3. Machine (10) selon la revendication précédente, **caractérisé en ce que** le dispositif (72) de distribution comporte uniquement deux chambres (76A, 76B) à volume variable.

4. Machine (10) selon la revendication précédente, **caractérisée en ce que** le dispositif (72) de commande est conçu pour déplacer simultané les deux pistons (78A, 78B) en sens opposées.

5. Machine (10) selon la revendication précédente, **caractérisé en ce que** le rapport de la variation de volume d'une des chambres (76A, 76B) à volume variable sur la distance de déplacement du piston (78A, 78B) associé est identique pour la première chambre (76A) à volume variable et pour la deuxième chambre (76B) à volume variable.

6. Machine (10) selon l'une quelconque des revendications 4 à 5, **caractérisé en ce que** le dispositif (94) de commande déplace le premier piston (78A) et le deuxième piston (78B) simultanément à la même vitesse dans deux sens opposés.

7. Machine (10) selon la revendication précédente, **caractérisé en ce que** les deux pistons (78A, 78B) sont liés mécaniquement en déplacement l'un à l'autre, le dispositif (94) de commande comportant un unique actionneur (96) commun aux deux pistons (78A, 78B).

8. Machine (10) selon la revendication précédente, **caractérisée en ce que** les deux pistons (78A, 78B) sont fixés ensemble.

9. Machine (10) selon l'une quelconque des revendications 2 à 6, **caractérisé en ce que** les deux pistons (78A, 78B) sont commandés indépendamment par deux actionneurs (96A, 96B) individuels.

10. Machine (10) selon la revendication précédente prise en combinaison avec la revendication 6, **caractérisé en ce que** les deux actionneurs (96A, 96B) individuels sont asservis l'un à l'autre.

11. Machine (10) selon l'une quelconque des revendications 2 à 10, **caractérisée en ce que** les chambres (76A, 76B) à volume variable sont raccordées en parallèle à la source (64) de liquide stérilisant, chacune par une vanne (88A, 88B) commandée associée.

12. Machine (10) selon l'une quelconque des revendications 2 à 10, **caractérisée en ce que** les chambres (76A, 76B) à volume variable sont raccordées en parallèle à la source (64) de liquide stérilisant par un clapet anti-retour qui autorise la circulation du liquide stérilisant uniquement depuis la source (64) de liquide stérilisant vers la chambre (76A, 76B) à volume variable associée.

13. Machine (10) selon l'une quelconque des revendications précédentes, **caractérisé en ce que** les chambres (76A, 76B) à volume variable sont raccordées en parallèle à un système (86) d'évacuation.

14. Machine (10) selon l'une quelconque des revendications 2 à 13, **caractérisée en ce que** les chambres (76A, 76B) à volume variable sont raccordées en parallèle à l'évaporateur (66), chacune par l'intermédiaire d'une vanne 90A, 90B) commandée associée.

15. Machine (10) selon l'une quelconque des revendications 13 ou 14, **caractérisé en ce que** chaque chambre (76A, 76B) à volume variable est raccordée au système (86) d'évacuation par l'intermédiaire d'une vanne (92A, 92B) commandée associée.

## Patentansprüche

1. Formmaschine (10) zum Formen von Behältern aus Vorformlingen aus thermoplastischem Material, wobei die Formmaschine (10) ein Blasnetz (26) aufweist, das Leitungen aufweist, die eine gesteuerte Quelle (24) von komprimiertem Blasgas mit mindestens einer Blasdüse (20) zum Ausbilden der Behälter durch Blasen verbinden, wobei die Formmaschine (10) eine Vorrichtung (46) zur Sterilisation des Blasnetzes (26) aufweist, die Folgendes aufweist:
- eine Quelle (64) von sterilisierender Flüssigkeit, die ein Oxidationsmittel aufweist;
**dadurch gekennzeichnet, dass** die Formmaschine ferner Folgendes umfasst:
- einen Verdampfer (66), in dem durch vollständiges Verdampfen der durch die Sterilisierungsflüssigkeitsquelle (64) bereitgestellten Sterilisierungsflüssigkeit sterilisierender Dampf erzeugt wird;
- eine Vorrichtung (72) zur Abgabe der Sterilisierungsflüssigkeit, die sich zwischen der Sterilisierungsflüssigkeitsquelle (64) und dem Verdampfer (66) befindet;
wobei die Abgabevorrichtung (72) so gestaltet ist, dass sie einen kontinuierlichen und stabilen Fluss von Sterilisierungsflüssigkeit abgibt.

2. Maschine (10) nach dem vorangehenden Anspruch, **dadurch gekennzeichnet, dass** die Abgabevorrichtung (72) Folgendes aufweist:
- eine erste Kammer (76A) mit variablem Volumen, die mit der Sterilisierungsflüssigkeitsquelle (64) und mit dem Verdampfer (66) in Verbindung steht, wobei das Volumen der ersten Kammer (76A) mit variablem Volumen gesteuert wird, indem ein erster beweglicher Kolben (78A) abwechselnd in einer Ansaugrichtung oder in einer entgegengesetzten Ausstoßrichtung bewegt wird;
- eine zweite Kammer (76B) mit variablem Volumen, die mit der Sterilisierungsflüssigkeitsquelle (64) und mit dem Verdampfer (66) in Verbindung steht, wobei das Volumen der zweiten Kammer (76B) mit variablem Volumen gesteuert wird, indem ein zweiter beweglicher Kolben (78B) abwechselnd in einer Ansaugrichtung oder in einer entgegengesetzten Ausstoßrichtung bewegt wird;
- eine Vorrichtung (94) zum Steuern der Bewegung des ersten Kolbens (78A) und des zweiten Kolbens (78B), so dass eine der beiden Kammern (76A, 76B) mit variablem Volumen die Sterilisierungsflüssigkeit ansaugt, während die andere der beiden Kammern (76B) mit variablem Volumen die Sterilisierungsflüssigkeit abgibt.

3. Maschine (10) nach dem vorangehenden Anspruch, **dadurch gekennzeichnet, dass** die Abgabevorrichtung (72) lediglich zwei Kammern (76A, 76B) mit variablem Volumen aufweist.

4. Maschine (10) nach dem vorangehenden Anspruch, **dadurch gekennzeichnet, dass** die Steuervorrichtung (72) so gestaltet ist, dass sie die beiden Kolben (78A, 78B) gleichzeitig in entgegengesetzte Richtungen bewegt.

5. Maschine (10) nach dem vorangehenden Anspruch, **dadurch gekennzeichnet, dass** das Verhältnis der Volumenänderung einer der Kammern (76A, 76B) mit variablem Volumen zum Bewegungsweg des zugehörigen Kolbens (78A, 78B) für die erste Kammer (76A) mit variablem Volumen und für die zweite Kammer (76B) mit variablem Volumen gleich ist.

6. Maschine (10) nach einem der Ansprüche 4 bis 5, **dadurch gekennzeichnet, dass** die Steuervorrichtung (94) den ersten Kolben (78A) und den zweiten Kolben (78B) gleichzeitig mit derselben Geschwindigkeit in zwei entgegengesetzte Richtungen bewegt.

7. Maschine (10) nach dem vorangehenden Anspruch, **dadurch gekennzeichnet, dass** die beiden Kolben (78A, 78B) mechanisch beweglich miteinander verbunden sind, wobei die Steuervorrichtung (94) einen einzigen gemeinsamen Aktuator (96) für beide Kolben (78A, 78B) aufweist.

8. Maschine (10) nach dem vorangehenden Anspruch, **dadurch gekennzeichnet, dass** die beiden Kolben (78A, 78B) aneinander befestigt sind.

9. Maschine (10) nach einem der Ansprüche 2 bis 6, **dadurch gekennzeichnet, dass** die beiden Kolben (78A, 78B) unabhängig voneinander durch zwei einzelne Aktuatoren (96A, 96B) gesteuert werden.

10. Maschine (10) nach dem vorangehenden Anspruch in Kombination mit Anspruch 6, **dadurch gekennzeichnet, dass** die beiden einzelnen Aktuatoren (96A, 96B) einander nachgeführt werden.

11. Maschine (10) nach einem der Ansprüche 2 bis 10, **dadurch gekennzeichnet, dass** die Kammern (76A, 76B) mit variablem Volumen jeweils über ein zugehöriges gesteuertes Ventil (88A, 88B) parallel mit der Sterilisierungsflüssigkeitsquelle (64) verbunden sind.

12. Maschine (10) nach einem der Ansprüche 2 bis 10, **dadurch gekennzeichnet, dass** die Kammern (76A, 76B) mit variablem Volumen über ein Rückschlagventil, das die Zirkulation der Sterilisierungsflüssigkeit nur von der Sterilisierungsflüssigkeitsquelle (64) zur zugehörigen Kammer (76A, 76B) mit variablem Volumen zulässt, parallel mit der Sterilisierungsflüssigkeitsquelle (64) verbunden sind.

13. Maschine (10) nach einem der vorangehenden Ansprüche, **dadurch gekennzeichnet, dass** die Kammern (76A, 76B) mit variablem Volumen parallel mit einem Abfuhrsystem (86) verbunden sind.

14. Maschine (10) nach einem der Ansprüche 2 bis 13, **dadurch gekennzeichnet, dass** die Kammern (76A, 76B) mit variablem Volumen jeweils über ein zugehöriges gesteuertes Ventil (90A, 90B) parallel mit dem Verdampfer (66) verbunden sind.

15. Maschine (10) nach einem der Ansprüche 13 oder 14, **dadurch gekennzeichnet, dass** jede Kammer (76A, 76B) mit variablem Volumen über ein zugehöriges gesteuertes Ventil (92A, 92B) mit dem Abfuhrsystem (86) verbunden ist.

## Claims

1. Shaping machine (10) for shaping containers from preforms made of thermoplastic material, the shaping machine (10) having a blowing network (26) that has ducts that connect a controlled source (24) of compressed blowing gas to at least one blowing nozzle (20) in order to shape the containers by blow-moulding, the shaping machine (10) having a device (46) for sterilizing the blowing network (26), said device having:
- a source (64) of sterilizing liquid having an oxidizing agent;
**characterized in that** the shaping machine further comprises:
- an evaporator (66) in which sterilizing vapour is produced by total evaporation of the sterilizing liquid provided by the source (64) of sterilizing liquid;
- a device (72) for dispensing the sterilizing liquid, said device being interposed between the source (64) of sterilizing liquid and the evaporator (66);
the dispensing device (72) being designed to deliver a continuous and stable flow rate of sterilizing liquid.

2. Machine (10) according to the preceding claim, **characterized in that** the dispensing device (72) has:
- a first variable-volume chamber (76A) communicating with the source (64) of sterilizing liquid and with the evaporator (66), the volume of the first variable-volume chamber (76A) being controlled by the movement of a first piston (78A) that can move alternately in an intake direction or in an opposite delivery direction;
- a second variable-volume chamber (76B) communicating with the source (64) of sterilizing liquid and with the evaporator (66), the volume of the second variable-volume chamber (76B) being controlled by the movement of a second piston (78B) that can move alternately in an intake direction or in an opposite delivery direction;
- a device (94) for controlling the movement of the first piston (78A) and of the second piston (78B) such that one of the two variable-volume chambers (76A, 76B) draws in the sterilizing liquid while the other of the two variable-volume chambers (76B) delivers the sterile liquid.

3. Machine (10) according to the preceding claim, **characterized in that** the dispensing device (72) has just two variable-volume chambers (76A, 76B).

4. Machine (10) according to the preceding claim, **characterized in that** the control device (72) is designed to simultaneously move the two pistons (78A, 78B) in opposite directions.

5. Machine (10) according to the preceding claim, **characterized in that** the ratio of the variation in volume of one of the variable-volume chambers (76A, 76B) to the distance by which the associated piston (78A, 78B) moves is identical for the first variable-volume chamber (76A) and for the second variable-volume chamber (76B).

6. Machine (10) according to either one of Claims 4 and 5, **characterized in that** the control device (94) moves the first piston (78A) and the second piston (78B) simultaneously at the same speed in two opposite directions.

7. Machine (10) according to the preceding claim, **characterized in that** the two pistons (78A, 78B) are mechanically connected in terms of movement to one another, the control device (94) having one single actuator (96) that is common to the two pistons (78A, 78B) .

8. Machine (10) according to the preceding claim, **characterized in that** the two pistons (78A, 78B) are fastened together.

9. Machine (10) according to any one of Claims 2 to 6, **characterized in that** the two pistons (78A, 78B) are independently controlled by two individual actuators (96A, 96B).

10. Machine (10) according to the preceding claim in combination with Claim 6, **characterized in that** the two individual actuators (96A, 96B) are automatically controlled by one another.

11. Machine (10) according to any one of Claims 2 to 10, **characterized in that** the variable-volume chambers (76A, 76B) are connected in parallel to the source (64) of sterilizing liquid, each by an associated controlled valve (88A, 88B).

12. Machine (10) according to any one of Claims 2 to 10, **characterized in that** the variable-volume chambers (76A, 76B) are connected in parallel to the source (64) of sterilizing liquid by a non-return valve that allows the sterilizing liquid to circulate solely from the source (64) of sterilizing liquid to the associated variable-volume chamber (76A, 76B).

13. Machine (10) according to any one of the preceding claims, **characterized in that** the variable-volume chambers (76A, 76B) are connected in parallel to a discharge system (86).

14. Machine (10) according to any one of Claims 2 to 13, **characterized in that** the variable-volume chambers (76A, 76B) are connected in parallel to the evaporator (66), each via an associated controlled valve (90A, 90B).

15. Machine (10) according to either one of Claims 13 and 14, **characterized in that** each variable-volume chamber (76A, 76B) is connected to the discharge system (86) via an associated controlled valve (92A, 92B).
